# EUROPÄISCHE PATENTSCHRIFT

(11) **EP 4 146 127 B1**
(45) Veröffentlichungstag und Bekanntmachung des Hinweises auf die Patenterteilung: **04.03.2026**
(21) Anmeldenummer: 22709199.8
(22) Anmeldetag: 31.01.2022
(51) Int. Cl.: A61F 5/01, A61F 5/058

(54) **ORTHESE**
ORTHOSIS
ORTHÈSE

(30) Priorität: 01.02.2021 DE 102021102282
(43) Veröffentlichungstag der Anmeldung: 15.03.2023
(73) Patentinhaber: Ferd. Hauber GmbH, 72622 Nürtingen (DE)
(72) Erfinder: LAITZSCH, Lukas, 10829 Berlin (DE); HORST, Christian, 73230 Kirchheim u. Teck (DE); BLANC, Stephan, 8404 Winterthur (CH); SCHMELTZPFENNING, Timo, 72074 Tübingen (DE)
(74) Vertreter: DREISS Patentanwälte PartG mbB
(86) Internationale Anmeldenummer: PCT/EP2022/052167
(87) Internationale Veröffentlichungsnummer: WO 2022/162208

(56) Entgegenhaltungen:
- EP-A1- 3 045 152
- EP-B1- 0 774 939
- WO-A1-2018/148702
- DE-A1- 102018 126 990
- US-A- 5 836 902
- US-A1- 2009 076 426

## Beschreibung

Die vorliegende Erfindung betrifft eine Orthese. Herkömmlicherweise werden Orthesen in einer Vielzahl verschiedener Größen angefertigt, **um** den Erfordernissen verschiedener Träger gerecht zu werden.

Dabei spielt insbesondere die Herstellung stabilitätsgebenden Strukturen, also stabilitätsgebenden Teilen der Orthese, bzw. deren Einzelteile (im Folgenden Strukturkomponenten) eine entscheidende Rolle. Häufig werden diese als Metall oder Kunststoffteile hergestellt und das Herstellen verschiedener Größen setzt den Einsatz verschiedener Werkzeuge voraus. Beispielsweise muss bei herkömmlichen Orthesen, deren Strukturkomponenten mittels Spritzgussverfahren hergestellt werden, für jede Größe der Orthese ein unterschiedliches Spritzgusswerkzeug verwendet werden. Dies führt zu hohen Werkzeugkosten. Auf der anderen Seite können die Unterschiede in den Abmessungen der Orthesen einzelner Größen nicht beliebig groß gewählt werden, da ansonsten ein korrekter Sitz der Orthese nicht gewährleistet werden kann.

US 2009 / 076 426 A1, EP 0 774 939 B1, DE 10 2018 126 990 A1, US 5 836 902 A und EP 3 045 152 A1 offenbaren jeweils eine Orthese mit Merkmalen des Anspruchs 1. WO 2018/148702 A1 offenbart eine Orthese mit den Merkmalen des Oberbegriffs des Anspruchs 1.

Der vorliegenden Erfindung liegt die Aufgabe zugrunde eine Orthese bereitzustellen, die von möglichst unterschiedlichen Trägern verwendet werden kann.

Zur Lösung dieses Problems schlägt die vorliegende Erfindung eine Orthese vor, die eine stabilitätsgebende Strukturkomponente mit hoher Anpassbarkeit und gleichzeitig ausreichender Stabilisierung aufweist. Eine derartige Orthese ist in Anspruch 1 definiert. Weitere Ausführungsvarianten der erfindungsgemäßen Orthese sind in der nachfolgenden Beschreibung sowie Unteransprüchen beschrieben.

Die Orthese umfasst eine stabilitätsgebende Strukturkomponente. Die Strukturkomponente umfasst ein festigkeitsgebendes Strukturmaterial bzw. kann aus diesem bestehen.

Die stabilitätsgebende Strukturkomponente weist über ihre gesamte Fläche eine unregelmäßige Gitterstruktur auf

Die unregelmäßige Gitterstruktur weist Stege aus festigkeitsgebendem Strukturmaterial auf und die Stege begrenzen unregelmäßig geformte strukturmaterialfreie Aussparungen. In stabilisierenden Bereichen können die Stege dicker ausgeführt sein, als in anpassbaren Bereichen.

Erfindungsgemäß ist der überwiegende Teil oder alle der Stege in stabilisierenden Bereichen mit einer wenigstens doppelt so dicken Materialstärke ausgebildet sein wie - ein überwiegender Teil oder alle der Stege in den anpassbaren Bereichen.

Die Strukturkomponente ist flächig ausgebildet. Mit flächig ist dabei eine Ausgestaltung der Strukturkomponente gemeint, in der die Stärke der Strukturkomponente in Richtung der lokalen Flächennormalen deutlich geringer ist, als die Dimensionierung der Strukturkomponente entlang ihrer flächigen Erstreckung. Mit einer flächigen Erstreckung ist dabei jedoch nicht die Erstreckung in einer geometrischen Ebene gemeint, sondern die Strukturkomponente kann eine dreidimensional geschwungene bzw. gekrümmte Form aufweisen. Typischerweise ist die Strukturkomponente komplementär zu der zu unterstützenden Gliedmaße bzw. der Körperform in einer Gelenksumgebung ausgebildet.

Die Strukturkomponente weist wenigstens eine in der flächigen Erstreckung liegende Stabilisierungsrichtung auf.

Quer zur Stabilisierungsrichtung ist die Strukturkomponente lokal (in einem Bereich der Strukturkomponente) stabilisierend biegestarr ausgebildet. Im angelegten Zustand der Orthese ist die Strukturkomponente derart angeordnet, dass die Stabilisierungsrichtung der Strukturkomponente entlang des Körpers des Trägers verläuft, so dass das zu stabilisierende Körperteil des Trägers durch die Strukturkomponente stabilisiert wird. Eine Biegung quer zur Stabilisierungsrichtung ist nur minimal möglich. Quer zur Stabilisierungsrichtung ist die Strukturkomponente also derartig starr ausgebildet, dass sie zur Stütze bzw. Stabilisierung einer Gliedmaße des Trägers der Orthese dient (im angelegten Zustand). Mit stabilisierend biegestarr in diesem Sinne ist eine Biegesteifigkeit gemeint, die eine gewisse Biegung zulassen kann, wie sie bei einer Schiene einer Orthese üblich ist, jedoch jedenfalls derart gering ist, dass die Stützende Funktion und Stabilisierung der Gliedmaße erfüllt ist.

Neben der Stabilisierungsrichtung weist die Strukturkomponente eine in der flächigen Erstreckung liegende Anpassungsrichtung auf. Quer zu der Anpassungsrichtung ist die Strukturkomponente lokal biegsam ausgebildet. Dabei ist die Strukturkomponente quer zur Anpassungsrichtung derart biegsam ausgebildet, dass sie durch Biegung quer zur Anpassungsrichtung an die Kontur einer Gliedmaße eines Trägers der Orthese in flächige Anlage bringbar ist. Die hierfür nötige Biegung kann beim Anlegen manuell erzielt werden. Bspw. kann die Orthese bzw. deren Strukturkomponente(n) durch entsprechende Befestigungsgurte fixiert und gleichzeitig durch Biegung an die lokale Körperform des Trägers angepasst werden. Die Befestigung und Anpassung mittels der Befestigungsgurte trägt auch zum festen Sitz der Orthese bei. Quer zur Anpassungsrichtung ist die Strukturkomponente zwar biegsam, sie bietet einer Biegung jedoch insbesondere einen gewissen Widerstand, sodass sie eine Spannung beim Anlegen aufrechterhält.

Die im Sinne dieser Erfindung diskutierten Biegungen der Strukturkomponenten beziehen sich auf eine Biegung in Richtung der lokalen Flächennormalen der Erstreckung der Strukturkomponente. Die lokale Stabilisierungsrichtung und die lokale Flächennormale spannen somit eine Stabilisierungsebene (eine Ebene im geometrischen Sinn) auf. Die Strukturkomponente ist in biegesteifen Bereichen derart biegesteif, dass ihr Querschnitt in dieser Stabilisierungsebene sich bei Belastung quasi nicht ändert. Die lokale Anpassungsrichtung und die lokale Flächennormale spannen eine Anpassungsebene (eine Ebene im geometrischen Sinn) auf. Die Strukturkomponente ist in anpassbaren Bereichen derart flexibel, dass ihr Querschnitt in dieser Anpassungsebene sich bei Belastung ändert und insbesondere an verschiedene Körperformen von unterschiedlichen Trägern anpassen lässt.

Die Orthese umfasst also wenigstens eine Strukturkomponente, die quer zu der Stabilisierungsrichtung lokal ausreichend steif für ein Stabilisieren eines Körperteils des Trägers ausgebildet ist und im Gegensatz hierzu quer zu der Anpassungsrichtung lokal derart flexibel ausgebildet ist, dass sie an verschiedene Körpergrößen anpassbar ist.

Beispielsweise kann hierdurch eine erfindungsgemäße Handgelenksorthese an unterschiedliche Handgrößen bzw. Armdurchmesser angepasst werden oder auch eine Knieorthese an unterschiedliche Beindurchmesser angepasst werden. Die Anpassung erfolgt hierbei durch die biegsame und flexible Ausgestaltung der Strukturkomponente quer zur Anpassungsrichtung beim Anlegen der Orthese bspw. durch Spannen und Befestigen mittels Befestigungsgurt oder Befestigungsgurten. Eine beispielsweise werkzeuggestützte Anpassung durch Justierung entsprechender Winkel und Variation eines Durchmessers ist bei der erfindungsgemäßen Orthese nicht nötig.

Die bereits eingangs beschriebene Gitterstruktur der Strukturkomponente stellt eine einfach herzustellende Möglichkeit zur Ausgestaltung der Strukturkomponente dar, mittels der zuverlässig die gewünschten Eigenschaften bereitgestellt werden können. Erfindungsgemäß wird durch eine gezielte Auswahl der Platzierung und Dimensionierung einzelner Stege und eine Variation der Anzahl der Stege eine lokale Einstellung der Steifigkeit der Strukturkomponente über ihre flächige Erstreckung erreicht.

Hierdurch werden gezielt einzelne Bereiche der Strukturkomponente biegesteif und andere Bereiche flexibel gestaltet.

. Verschiedene lokale Steifigkeiten und Flexibilitätsgrade sind einfach realisierbar.

Die Gitterstruktur weist insbesondere einen geschlossenen Rand auf. Es enden also keine Stege in Richtung ihrer Längserstreckung im Rand. Der Rand wird vielmehr durch einen umlaufenden Steg gebildet.

Die materialfreien Aussparungen weisen insbesondere eine Vielzahl unterschiedlicher Formen auf, insbesondere wenigstens 3, insbesondere 5, insbesondere 7, verschiedene Formen an Aussparungen an einer Strukturkomponente. Insbesondere sind die Aussparungen überwiegend oder ausschließlich mit verrundeten Ecken ausgebildet. Die Stege können sich in Richtung von Verbindungspunkten mit weiteren Stegen in ihrer Dicke in der flächigen Erstreckung der Strukturkomponente verdicken. Insbesondere gibt in einer Strukturkomponente verschiedenartige Verbindungspunkte, insbesondere Verbindungspunkte in denen ein Steg auf einen gerade weiterverlaufenden Steg trifft, Verbindungspunkte in denen mehrere Stege auf einen gerade weiterverlaufenden Steg treffen und Verbindungspunkte in denen mehrere (insbesondere 3 bis 6, insbesondere bis 5) Stege aus unterschiedlichen Richtungen aufeinandertreffen. Die Strukturkomponente kann Verbindungspunkte umfassen in denen sich 2 gerade weiterverlaufende Stege kreuzen. Die unterschiedlichen Verbindungspunkte können in unterschiedlichen Kombinationen in den Strukturkomponenten vorhanden sein.

Die Strukturkomponente kann insbesondere einen oder mehrere stabilisierende Bereiche aufweisen und weist erfindungsgemäß mehrere anpassbare Bereiche auf. Die Strukturkomponente umfasst anpassbare Bereiche, die jeweils neben einem stabilisierenden Bereich angeordnet sind beim Blick entlang der Stabilisierungsrichtung in diesem stabilisierenden Bereich.

Die Strukturkomponente kann insbesondere einen teilweise flexiblen Bereich aufweisen in einem derartigen teilweise flexiblen Bereich weist die Strukturkomponente in einem Winkel von wenigstens 45° zueinander, insbesondere von wenigstens 65°, insbesondere von wenigstens 80° zueinander verlaufende Stabilisierungsrichtungen und Anpassungsrichtungen auf.

Die Stabilisierungsrichtungen geben insbesondere die Richtungen mit der größten Steifigkeit an und die Anpassungsrichtungen insbesondere mit der größten Flexibilität.

Beispielsweise kann in dem stabilisierenden Bereich um die Stabilisierungsrichtung herum eine erhöhte Anzahl an Stegen vorgesehen sein, um eine stabilisierende Wirkung zu erreichen. Erfindungsgemäß werden in dem stabilisierenden Bereich Stege mit höherer Materialstärke, insbesondere quer zur Stabilisierungsrichtung, vorgesehen.

Insbesondere können die Stege auch überwiegend in Stabilisierungsrichtung erstreckt ausgebildet sein.

Entsprechend können in dem anpassbaren Bereich die Stege überwiegend quer zur Anpassungsrichtung erstreckt verlaufen. Die Stege könne hierzu in überwiegender Anzahl quer zur Anpassungsrichtung verlaufen oder ihre Richtung ist überwiegend quer zur Anpassungsrichtung (in einem geringen Winkel zur Anpassungsrichtung). Die Stege in dem anpassbaren Bereich weisen eine geringere Materialstärke als die Stege in dem stabilisierenden Bereich auf.

Die räumliche Dichte der Stege kann in dem anpassbaren Bereich geringer gewählt sein, als in dem stabilisierenden Bereich. Die verschiedenen Wege eine Anpassbarkeit bzw. Flexibilität und eine Steifheit zu erreichen könne einzeln oder auch kombiniert miteinander angewendet sein.

Die Orthese kann wie erwähnt einen oder mehrere Befestigungsgurte umfassen. Der Befestigungsgurt ist insbesondere flächig ausgebildet. In seiner Breite und in seiner Länge kann er eine wesentlich größere Erstreckung aufweisen als in seiner zu diesen beiden Richtungen Materialdicke. Er kann aus einem biegeschlaffen Material gefertigt sein. Er kann unelastisch ausgebildet sein. Insbesondere kann die Strukturkomponente mit wenigstens einem Befestigungsgurt verbunden sein. Der Befestigungsgurt kann unlösbar an der Strukturkomponente befestigt sein. Insbesondere kann der Befestigungsgurt zwar gegenüber der Strukturkomponente verschiebbar jedoch unlösbar mit dieser verbunden befestigt sein. Der Befestigungsgurt oder die Befestigungsgurte können auch mittels einer Haken-Ösen-Materialverbindung auf sich selbst festlegbar ausgebildet sein und durch eine Öse oder einen Schlitz oder andere Ausnehmung in der Strukturkomponente bzw. der stabilitätsgebenden Struktur der Orthese geführt sein. Der Befestigungsgurt kann einen Ausfädelstop, insbesondere in Form einer unlösbar mit dem biegeschlaffen Material des Befestigungsgurts verbundenen Kunststoffkomponente. Insbesondere kann der Ausfädelstop ein Kunststoffring sein, der insbesondere durch Ultraschallschweißung mit dem Material des Befestigungsgurts verbunden ist.

Der Befestigungsgurt kann in einer festgelegten Erstreckungsrichtung an der Strukturkomponente befestigt sein und zur zirkulären Umschlingung der durch die Orthese zu stabilisierenden Gliedmaße des Trägers der Orthese ausgebildet sein. Insbesondere kann vorgesehen sein, dass der Befestigungsgurt mit einem seiner Enden an einer Durchführung an der Strukturkomponente befestigt ist und von dieser abgehend erstreckt ist. Weiter kann ein dem befestigten Ende gegenüberliegendes freies Ende des Befestigungsgurts durch eine weitere Durchführung geführt sein. Das freie Ende des Befestigungsgurts kann zur Befestigung der Orthese mittels einer Haken-Ösen Verbindung, beispielsweise Klettverschluss, auf sich selbst festgelegt werden. Die Umschlingung der Gliedmaße kann durch den Befestigungsgurt selbst (Befestigungsgurt umschlingt den gesamten Umfang der Gliedmaße) oder durch den Befestigungsgurt und wenigstens eine Strukturkomponente gebildet sein.

Die Orthese kann wenigstens zwei Strukturkomponenten umfassen, die jeweils in sich einstückig ausgebildet sind. Die wenigstens zwei Strukturkomponenten können getrennt voneinander oder miteinander verbunden an der Orthese angeordnet sein.

Die Strukturkomponenten können beispielsweise über ein Gelenk schwenkbar miteinander verbunden sind. Typischerweise erlaubt eine derartige gelenkige Verbindung zwischen den Strukturkomponenten eine Veränderung der Stellung der Strukturkomponenten quer zu einer jeweiligen Stabilisierungsrichtung der Strukturkomponenten.

Die wenigstens zwei Strukturkomponenten können über eine Verbindungseinrichtung in zueinander festgelegter Position und Ausrichtung miteinander verbunden sein und gegenüber einander fixiert sein (in zueinander relativer Position und Ausrichtung festgelegt sein).

Die Orthese kann eine biegeschlaffe Polsterkomponente auf einer zur Kontaktierung der Gliedmaße des Trägers vorgesehenen Seite der Strukturkomponente umfassen. Die Polsterkomponente kann mit der Strukturkomponente lösbar oder unlösbar verbunden sein. Insbesondere kann die Polsterkomponente in die Strukturkomponente einsetzbar sein. Die Strukturkomponente kann beispielsweise lochartige Durchbrüche aufweisen und die Polsterkomponente hierzu komplementär ausgebildete Clipsverbindungen.

Die Strukturkomponente ist als einstückiges Spritzgussteile hergestellt. Insbesondere kann vorgesehen sein, dass die Strukturkomponente hinterschnittfrei ausgebildet ist, um ein einfaches Entformen zu gewährleisten.

In einer nicht beanspruchten Ausgestaltung kann die Orthese eine Sprunggelenksorthese sein. Die Sprunggelenksorthese weist distalseitig vorzugsweise zwei zur jeweils seitlichen und fußsohlenseitigen Kontaktierung des Fußes ausgebildete Schalenelemente auf, die einen zur Anordnung parallel zur Fußsohle eines Trägers ausgerichteten Verbindungsabschnitt aufweisen und einen in proximaler Richtung abgehend erstreckten Stützabschnitt. An den Stützabschnitt schließt sich vorzugsweise ein Gelenk an. An dieses Gelenk schließt sich an jedes der Schalenelemente eine Strukturkomponente an, welche durch das Gelenk schwenkbar mit dem jeweiligen Schalenelement verbunden ist. Die jeweiligen Strukturkomponenten sind vorzugsweise gekrümmt ausgebildet, sodass sie der Kontur des Unterschenkels eines Trägers im Wesentlichen folgen. Weiter ist die Strukturkomponente vorzugweise derart ausgebildet, dass sie in dieser Krümmung flexibel anpassbar ist, also an verschiedene Unterschenkelumfänge eines Trägers anpassbar gestaltet ist. Gleichzeitig ist die Strukturkomponente jedoch derart starr ausgebildet, dass eine Biegung quer zur distal proximalen Achse nicht bzw. nur minimal möglich ist. Um auch an verschiedene Fußbreiten eines Trägers möglichst flexibel angepasst werden zu können, sind die beiden Schalenelemente über eine lösbare Verbindung, welche vorzugsweise in Form einer Haken-Ösen Materialverbindung (Klettverschluss) ausgebildet ist, miteinander lösbar verbindbar. Hierdurch kann die relative Position der beiden Schalenelemente an die Fußbreite des Trägers angepasst werden, indem die Überlappung der beiden Verbindungsabschnitte verringert oder vergrößert wird. Hierdurch kann auch das Gelenk, das die Schalenelemente mit den jeweiligen Strukturkomponenten verbindet, möglichst nahe am Sprunggelenk platziert werden.

In einer nicht beanspruchten Ausgestaltung ist die Orthese insbesondere eine Sprunggelenksorthese und weist distalseitig zwei zur jeweils seitlichen Kontaktierung des Fußes eines Trägers ausgebildete Schalenelemente auf, die in variabler Position gegenüber einander festlegbar sind, wobei die Schalenelemente über ein jeweiliges proximal an den Schalenelementen angeordnetes Gelenk schwenkbar mit einer jeweiligen Strukturkomponente verbunden sind, wobei sich die Strukturkomponente in proximaler Richtung von dem Gelenk aus erstreckt. Die sich in proximaler Richtung von dem Gelenk aus erstreckte Strukturkomponente kann einen mittigen in distal-proximal-verlaufender Richtung erstreckten stabilisierenden Bereich umfassen, dessen Stabilisierungsrichtung kann in distal-proximal-verlaufender Richtung erstreckt sein. Die Orthese kann weiter beim Blick in distal-proximal-verlaufender Richtung seitlich von dem stabilisierenden Bereich angeordnete anpassbare Bereiche umfassen. Deren Anpassungsrichtung kann in Umfangsrichtung des Unterschenkels des Trägers im angelegten Zustand verlaufen.

Im Sinne der vorliegenden Erfindung ist die Orthese eine Handgelenksorthese.

Eine derartige Handgelenksorthese weißt einen Handgelenksstützabschnitt, der durch eine erste Strukturkomponente gebildet ist, auf. Weiter kann die Handgelenksorthese eine Fingerauflage, die als eine zweite Strukturkomponente ausgebildet sein kann, aufweisen. Weiter kann die Handgelenksorthese alternativ oder zusätzlich hierzu auch eine Daumenauflage umfassen, die als eine dritte Strukturkomponente ausgebildet sein kann.

Es ist auch möglich, dass die Handgelenksorthese lediglich einen Handgelenksstützabschnitt und die zugehörige Daumenauflage umfasst. Die einzelnen Finger sind in diesem Fall dann frei beweglich und werden durch die Handgelenksorthese nicht gestützt. Wie bereits gesagt können die beschriebenen Einheiten (Handgelenksstützabschnitt, Fingerauflage, Daumenauflage) der Handgelenksorthese als einzelne Strukturkomponenten ausgebildet sein, die miteinander verbunden werden bzw. sind, um eine stabilitätsgebende Struktur zu bilden. Ebenso ist möglich die einzelnen Einheiten (Handgelenksstützabschnitt, Fingerauflage, Daumenauflage) gemeinsam in einer Strukturkomponente zu realisieren.

Die den Handgelenksstützabschnitt bildende Strukturkomponente bzw. der Teil einer Strukturkomponente, der den Handgelenksstützabschnitt bildet, weißt einen von proximal nach distal erstreckten stabilisierenden Bereich auf, der sich im vorgesehenen angelegten Zustand bis über das Handgelenk erstreckt (aus proximaler Richtung kommend). Beim Blick in distaler Richtung links und rechts des stabilisierenden Bereichs sind in einer Umfangsrichtung gekrümmte anpassbare Bereiche angeordnet, die flexibel an den Armdurchmesser des Trägers anpassbar sind. Die unterschiedlichen Flexibilitäten bzw. Steifigkeiten oder Anpassbarkeiten der Strukturkomponenten bzw. der stabilitätsgebenden Struktur sind durch unterschiedliche Durchmesser von Stegen der Gitterstruktur der Strukturkomponenten bzw. stabilitätsgebenden Struktur realisiert. In dem von proximal nach distal erstreckten stabilisierenden Bereich verläuft die Stabilisierungsrichtung von proximal nach distal in der Fläche der Strukturkomponente. Die Strukturkomponente ist derart ausgebildet, dass sie im angelegten Zustand auf Seiten der Handinnenfläche angeordnet ist. Je seitlich des stabilisierenden Bereichs sind gekrümmte Anpassungsbereiche vorgesehen, die sich in ihrer Krümmung anpassen lassen, um an den Armdurchmesser des Trägers angepasst zu werden. In Armumfangrichtung weisen der stabilisierende Bereich und die gekrümmten Anpassungsbereiche insbesondere eine in etwa gleiche Erstreckung auf, wobei die Anpassungsbereiche jeweils eine höchstens 1,5, insbesondere höchstens 1,3, mal so große Erstreckung in Armumfangrichtung aufweisen. Dies bezieht sich auf den vom Handgelenk nach proximal erstreckten Teil der Strukturkomponente im angelegten Zustand.

In einer nicht beanspruchten Ausgestaltung kann die Orthese auch eine Knieorthese sein. Die Knieorthese weist eine stabilitätsgebende Struktur auf, die mehrere gelenkig miteinander verbundene Strukturkomponenten umfasst.

Ein proximaler Abschnitt der stabilitätsgebenden Struktur ist beidseitig auf Höhe des Kniegelenks (im angelegten Zustand) schwenkbar gelenkig mit einem distalen Abschnitt verbunden. Sowohl der proximale als auch der distale Abschnitt können jeweils wiederum wenigstens zwei Strukturkomponenten umfassen. Die Strukturkomponenten können insbesondere über ein distales und proximales Gelenk in ihrer Stellung zueinander anpassbar sein. Das proximale und das distale Gelenk erlauben dabei eine Schwenkbewegung um eine Achse, die orthogonal verläuft zur Knieschwenkachse bzw. zur Schwenkachse der Gelenke auf Höhe des Knies, die den proximalen Abschnitt mit dem distalen Abschnitt verbinden.

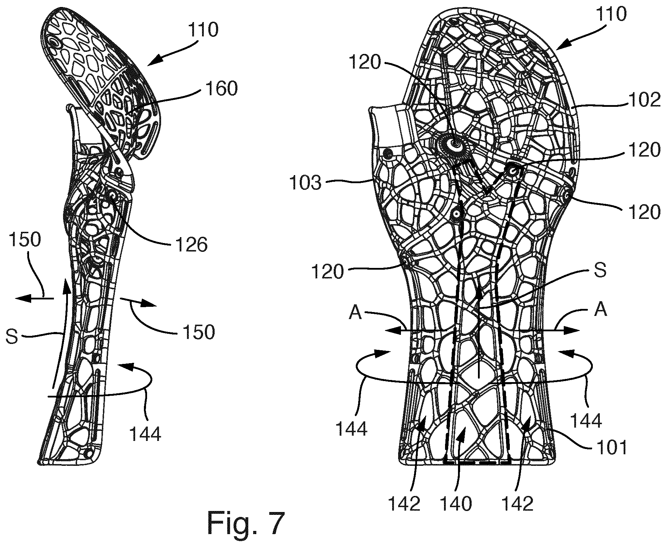

Durch diese Gelenke kann die Neigung der Abschnitte, der Strukturkomponenten, die parallel zum Bein des Trägers verlaufen, angepasst werden. Also die Neigung der proximal-distal-verlaufenden Abschnitte der Strukturkomponenten. Um diese Neigungsanpassung strukturell kompensieren zu können weisen die jeweiligen Strukturkomponenten einen biegsam anpassbaren Bereich auf, der durch einen stabilisierenden Bereich von dem jeweiligen Gelenk auf Höhe des Kniegelenks beabstandet ist. Die Strukturkomponenten können die oben beschriebene gitterartige Struktur mit Stegen und strukturmaterialfreie Aussparungen aufweisen. Die Flexibilität in dem anpassbaren Bereich kann insbesondere durch eine erhöhte Beabstandung und/oder dünnere Ausführung der Stege bedingt sein. Ebenfalls kann die Biegsamkeit in dem anpassbaren Bereich dadurch bewirkt sein, dass die Stege insgesamt eine Ausrichtung aufweisen, die mehr in Richtung einer Biegeachse, um die die Strukturkomponente in dem anpassbaren Bereich biegsam ist, verläuft als in dem stabilisierenden Bereich. Die drei Mechanismen zur Erreichung eines höheren oder reduzierten Flexibilitätsgrads können auch überlagert angewandt sein. Die Erfindung wird im Folgenden anhand der Figuren näher beschrieben, wobei gleiche oder funktional gleiche Elemente ggf. lediglich einmal mit Bezugszeichen versehen sind. Ausgestaltungen, die unter die beanspruchte Erfindung fallen sind in den Figuren 7-14 dargestellt. Die weiteren Figuren zeigen nicht beanspruchte Ausgestaltungen. Es zeigen:
Figur 1 eine Sprunggelenksorthese in einer Vorderansicht;
Figur 2 die Sprunggelenksorthese in einer Rückansicht;
Figur 3 die Sprunggelenksorthese in einer Seitenansicht;
Figur 4 einen Teil der Sprunggelenksorthese in einer perspektivischen Darstellung;
Figur 5 einen Teil der Sprunggelenksorthese in einer perspektivischen Darstellung;
Figur 6 einen Teil der Sprunggelenksorthese;
Figur 7 mehrere miteinander verbundene Strukturkomponenten einer Handgelenksorthese in weiteren Ansichten;
Figur 8 die Strukturkomponenten der Handgelenksorthese in einer weiteren Ansicht und entlang einer Schnittlinie B-B;
Figur 9 die Strukturkomponenten der Handgelenksorthese in einer weiteren Ansicht und entlang einer Schnittlinie A-A;
Figur 10 die Strukturkomponenten der Handgelenksorthese in einer weiteren Ansicht in voneinander losgelöstem Zustand;
Figur 11 eine der Strukturkomponenten der Handgelenksorthese im Detail sowie mehre Schnittlinien;
Figur 12 Schnittdarstellung der Strukturkomponente aus Figur 11;
Figur 13 Schnittdarstellung der Strukturkomponente aus Figur 11;
Figur 14 die Strukturkomponente der Handgelenksorthese mit einem an ihr angebrachten Befestigungsgurt;
Figur 15 die Knieorthese in einer Vorderansicht;
Figur 16 die Knieorthese in einer Seitenansicht;
Figur 17 die Knieorthese in einer Rückansicht;
Figur 18 die Knieorthese in einer Seitenansicht;
Figur 19 die Knieorthese in einer Seitenansicht;
Figur 20 die Knieorthese in einer Seitenansicht;
Figur 21 die Knieorthese mit eingezeichneten Biegerichtungen; und
Figur 22 die Knieorthese mit eingezeichneten Biegerichtungen.

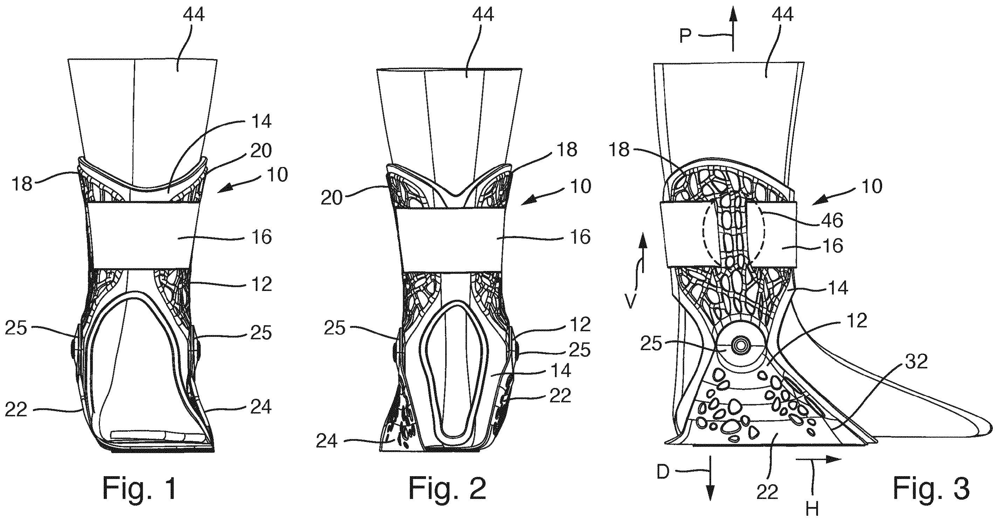

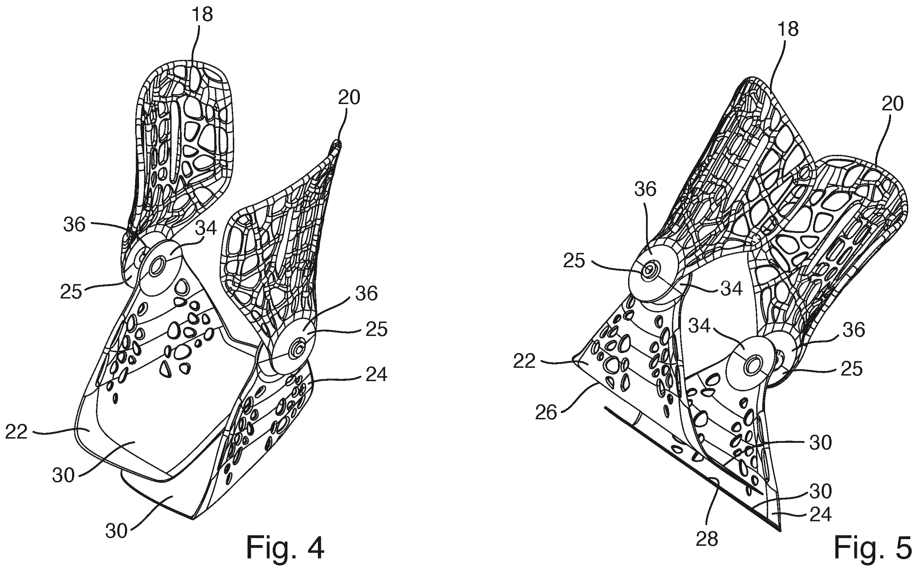

Figur 1 zeigt eine Orthese 10, die als Sprunggelenksorthese 10 ausgebildet ist, in einer Vorderansicht. Die Sprunggelenksorthese 10 umfasst eine stabilitätsgebende Struktur 12, eine Polsterkomponente 14 sowie einen Befestigungsgurt 16. Die Sprunggelenksorthese 10 ist in Figur 2 in einer Rückansicht gezeigt und in Figur 3 in einer Seitenansicht. In den Figuren 4 und 5 ist die stabilitätsgebende Struktur 12 der Sprunggelenksorthese 10 jeweils ohne die Polsterkomponente 14 und den Befestigungsgurt 16 gezeigt. Der Befestigungsgurt 16 ist zur zirkulären Umschlingung eines Körperteils, vorliegend eines Beins 44 des Trägers ausgebildet. Hierfür wird er durch eine Schlaufe 46 der stabilitätsgebenden Struktur 12 hindurchgeführt.

Die stabilitätsgebende Struktur 12 umfasst eine erste Strukturkomponente 18 und eine zweite Strukturkomponente 20. Die beiden Strukturkomponenten 18, 20 sind jeweils mit einem ersten und zweiten Schalenelement 22, 24 über ein jeweiliges Gelenk 25 schwenkbar gelenkig verbunden.

Die beiden Schalenelemente 22, 24 sind über eine Haken-Ösen Verbindung in einer gegenüber einander fixierten Position festlegbar. Hierzu weist das erste Schaltelement 22 an einer Unterseite 26 und das Schalenelement 24 an einer Oberseite 28 eines jeweiligen Verbindungsabschnitts 30 ein Haken-Ösen-Material (Klettverschluss) auf. In der Trageposition der Sprunggelenksorthese 10 bei einem stehenden Verwender, wie sie beispielsweise in Figuren 1-3 gezeigt ist, sind die Verbindungsabschnitte 30 jeweils in der Horizontalebene bzw. in horizontaler Richtung H erstreckt und in etwa rechtwinklig zu Stützabschnitten 32 der jeweiligen Schalenelemente 24 ausgerichtet. Die Stützabschnitte 32 erstrecken sich in etwa in vertikaler Richtung V bzw. von dem jeweiligen Gelenk 25 in proximaler Richtung P abgehend. Bezogen auf die Körperrichtungen befinden sich die Verbindungsabschnitte 30 am distalen Ende (distale Richtung D) der Schalenelemente 24 und die Stützabschnitt 32 erstrecken sich von diesen in proximaler Richtung **P.** Am proximalen Ende der Schalenelemente 24 sind die Gelenke 25 angeordnet. Entsprechend sind die Gelenke 25 am distalen Ende der Strukturkomponenten 18 und 20 angeordnet. Jeweils eine körpernahe Komponente 34 des jeweiligen Gelenks 25 ist an dem jeweiligen Schalenelement 24 angeordnet und eine körperabgewandte Komponente 36 des Gelenks 25 an der jeweiligen Strukturkomponente 18 bzw. 20.

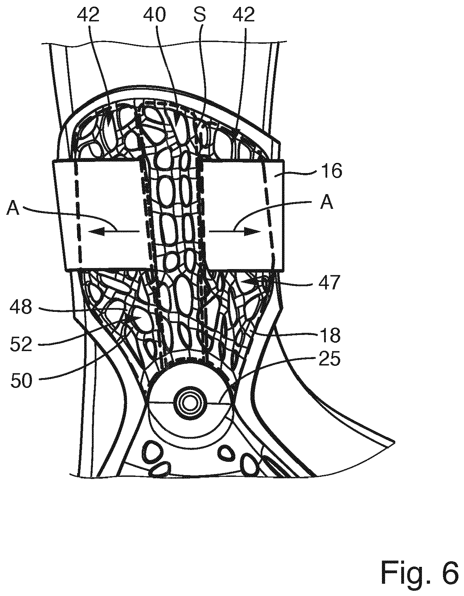

In Figur 6 ist die Strukturkomponente 18 im Detail vergrößert gezeigt. Die Strukturkomponente 18 weist einen stabilisierenden Bereich 40 auf, der durch eine gepunktete Linie umrandet ist. Eine Stabilisierungsrichtung S ist durch einen Pfeil gekennzeichnet. Beim Blick entlang der Stabilisierungsrichtung S sind jeweils seitlich neben dem stabilisierenden Bereich 40 anpassbare Bereiche 42 angeordnet. In den jeweiligen anpassbaren Bereichen 42 sind jeweilige Anpassungsrichtungen A durch entsprechende Pfeile gekennzeichnet. Die Stabilisierungsrichtung S und die Anpassungsrichtung A liegen jeweils in der flächigen Erstreckung der Strukturkomponente 18.

Quer zu der Stabilisierungsrichtung S ist die Strukturkomponente 18 stabilisierend biegestarr ausgebildet. Quer zu der Anpassungsrichtung A hingegen ist die Strukturkomponente 18 lokal biegsam ausgebildet. Die beiden anpassbaren Bereiche 42 können in ihrer Krümmung **um** ein Bein 44 eines Trägers variiert werden. Die Sprunggelenksorthese 10 kann hierdurch an die Kontur des Beins 44 angepasst werden. Die Sprunggelenksorthese 10 kann beispielsweise ausgeweitet werden, **um** an einem Bein 44 mit größerem Durchmesser angelegt werden.

Über ihre gesamte Fläche weist die Strukturkomponente 18 eine unregelmäßige Gitterstruktur 47 auf. Die unregelmäßige Gitterstruktur 47 umfasst Stege 48 aus festigkeitsgebendem Strukturmaterial. Die Stege 48 begrenzen unregelmäßig geformte strukturmaterialfreie Aussparungen 52.

Im stabilisierenden Bereich 40 sind die Stege 48 dicker ausgeführt, als in den anpassbaren Bereichen 42. Insbesondere ist der überwiegende Teil der Stege 48 im stabilisierenden Bereich 40 mit einer wenigstens doppelt so dicken Materialstärke ausgebildet als ein überwiegender Teil der Stege 48 in den anpassbaren Bereichen 42.

Figur 7 zeigt mehrere miteinander verbundene Strukturkomponenten 101, 102 und 103, die eine stabilitätsgebenden Struktur 112 einer erfindungsgemäßen Handgelenksorthese 110 bilden. Links in Figur 7 ist eine seitliche Ansicht der stabilitätsgebenden Struktur 112 gezeigt und rechts ist eine Ansicht auf die stabilitätsgebenden Struktur 112 gezeigt, beim Blick auf die der Fläche 160 zur Kontaktierung der Hand abgewandten Seite.

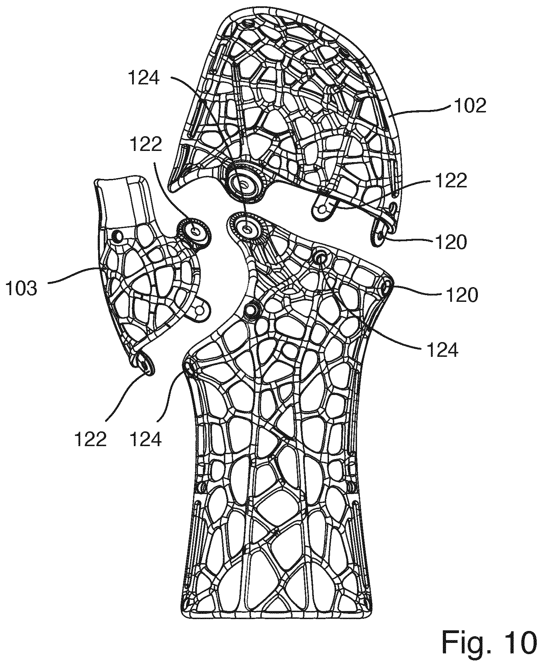

Die erste Strukturkomponente 101 ist zur Stabilisierung des Handgelenks eines Trägers ausgebildet. An die erste Strukturkomponente 101 ist eine zweite Strukturkomponente 102, die als Fingerauflage ausgebildet ist, angefügt. Ebenso ist eine dritte Strukturkomponente 103 (Daumenauflage) mit den beiden anderen Strukturkomponenten 101,102 verbunden. Die Strukturkomponenten 101,203 sind über Verbindungseinrichtungen 120 miteinander verbunden. In Figur 10 ist der Aufbau der vorliegend in diesem Beispiel verwendeten Verbindungseinrichtungen 120 gut zu erkennen. Die zweite und dritte Strukturkomponente 102, 103 weisen jeweils Fortsätze 122 auf, die im verbundenen Zustand, mit entsprechenden Ausnehmungen 124 überlappend angeordnet sind. Durch eine Niete, die einen Teil der Verbindungseinrichtungen 20 bildet, oder ein anderes Fixierungselement 126 sind die einzelnen Strukturkomponenten 101, 102, 103 im miteinander verbundenen Zustand aneinander befestigt.

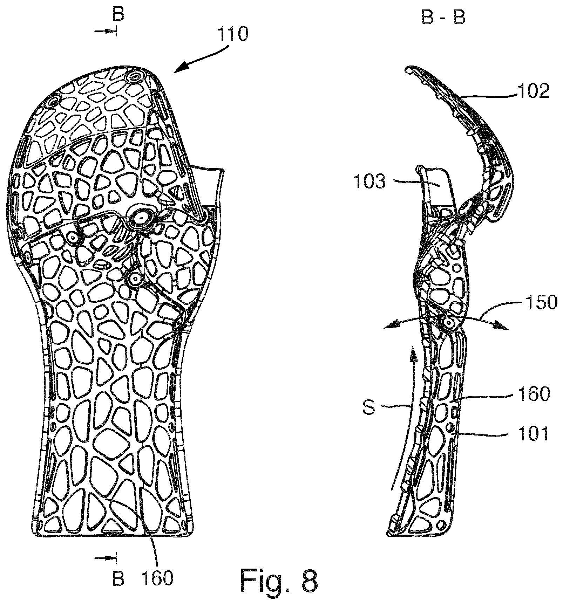

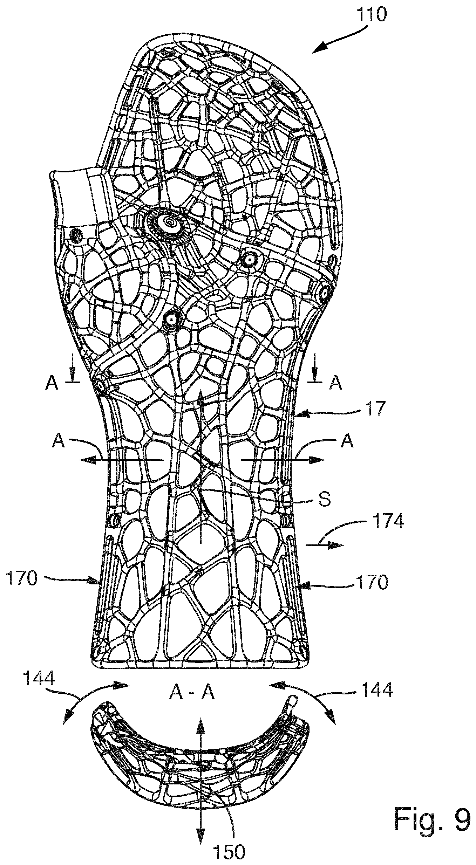

Die erste Strukturkomponente 101 erfüllt die Hauptfunktion der stabilitätsgebenden Struktur 112. Sie stabilisiert das Handgelenk des Trägers. Die Stabilisierungsrichtung S sowie die Anpassungsrichtung A sind an dieser Strukturkomponente 101 illustriert. In den Anpassungsbereichen 142, die jeweils beim Blick entlang der Stabilisierungsrichtung S neben dem stabilisierenden Bereich 140 angeordnet sind, ist die Strukturkomponente 101 flexibel in ihrer Krümmung anpassbar, was durch die gekrümmten Pfeile 144 illustriert ist. In dem stabilisierenden Bereich 140 ist die Strukturkomponente 101 quer zu der Stabilisierungsrichtung S stabilisierend biegestarr ausgebildet. In der linken Darstellung von Figur 7 sind die Richtungen, orthogonal zur Stabilisierungsrichtung S, in denen eine Biegung der Orthese nur minimal möglich ist, mit Pfeilen 150 gekennzeichnet. Die Stabilisierungsrichtung S sowie die Richtung, in der keine oder nur geringe Biegung der Strukturkomponente 101 möglich ist sind ebenso in den Figuren 8 und 9 illustriert. Gleiches gilt für die Anpassungsrichtung A und die Biegungsrichtungen der Strukturkomponente 101.

In Figur 8 ist die stabilitätsgebende Struktur 112 aus Figur 7 in einer Darstellung (links) gezeigt, beim Blick auf die zur Aufnahme der Hand vorgesehenen Fläche. Figur 8 zeigt weiter einen Schnitt entlang der Linie B-B durch die stabilitätsgebende Struktur 112. In Figur 9 ist eine Darstellung entsprechend der rechten Darstellung aus Figur 7 gezeigt sowie ein Schnitt entlang der Linie A-A, welche im unteren Bereich der Figur 9 gezeigt ist. Figur 9 illustriert anschaulich, wie eine Unbiegsamkeit bzw. minimale Biegsamkeit entlang des Pfeils 150 eine stabilisierende Wirkung auf das Handgelenk des Trägers ausüben kann und durch eine Biegsamkeit der stabilitätsgebenden Struktur 112 in Richtung der Pfeile 144 eine Anpassung bezüglich der Dicke des Arms des Trägers ermöglicht wird.

Über ihre gesamte Fläche weist die Strukturkomponente 101 eine unregelmäßige Gitterstruktur 147 auf. Die unregelmäßige Gitterstruktur 147 umfasst Stege 148 aus festigkeitsgebendem Strukturmaterial. Die Stege 148 begrenzen unregelmäßig geformte strukturmaterialfreie Aussparungen 152.

Wie in Figur 9 gezeigt, weist die stabilitätsgebende Struktur 112 mehrere Schlitze 170 und 172 auf, die zur Durchführung eines entsprechenden Befestigungsgurts ausgebildet sind. Auf Höhe der Schlitze 170 sind zwei gegenüber voneinander angeordnete Schlitze 170 vorgesehen. Ein Befestigungsgurt 116 kann in einem der Schlitze in einer festgelegten Erstreckungsrichtung 174 befestigt werden kann. Der Befestigungsgurt 116 wird **um** den Arm des Trägers geführt und durch den gegenüberliegenden Schlitz 170 geführt und von dort auf sich selbst zurückgeführt. Mittels an dem Befestigungsgurt 116 angebrachter Klettverbindungen kann so die stabilitätsgebende Struktur 112 an die Körperform des Trägers ideal angepasst werden. An dem Schlitz 172 ist ebenso die Befestigung eines Befestigungsgurts 116 vorgesehen. Der Befestigungsgurt 116 an diesem einzelnen Schlitz 172 ist jedoch zur Umschlingung des Arms des Trägers vorgesehen, der Befestigungsgurt 116 wird so auf sich selbst zurückgeführt.

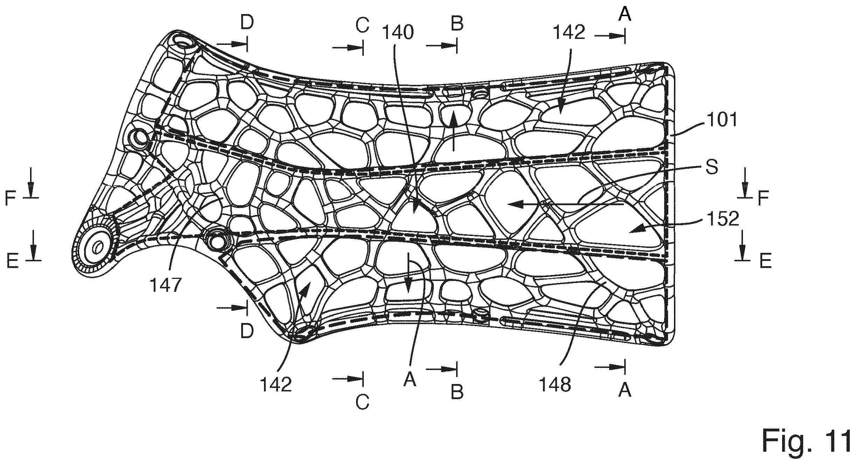

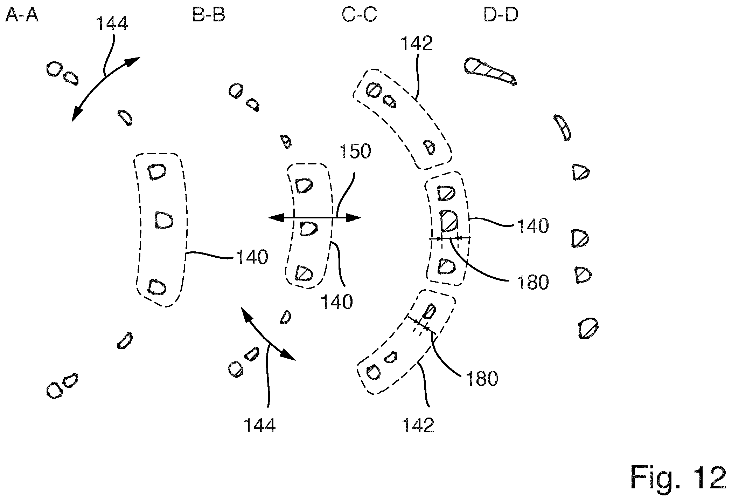

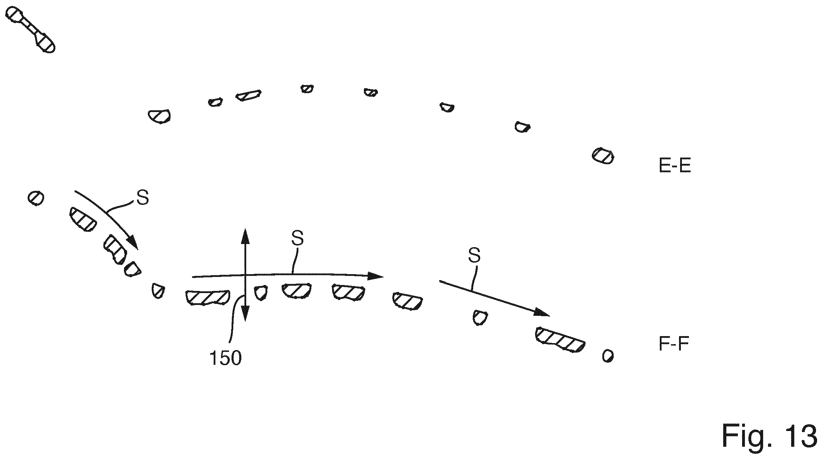

In Figur 11 ist die Strukturkomponente 101 vereinzelt gezeigt und es sind mehrere Schnittlinien dargestellt, deren Schnitte in den Figuren 12 und 13 gezeigt sind. In den Figuren 12 und 13 ist gut ersichtlich, dass die Strukturkomponente 101 Stege 148 mit einer unterschiedlichen Materialstärke 180 aufweisen.

Im stabilisierenden Bereich 140 sind die Stege 148 dicker ausgeführt, als in den anpassbaren Bereichen 142.

Der überwiegende Teil der Stege 148 im stabilisierenden Bereich 140 ist mit einer wenigstens doppelt so dicken Materialstärke 180 ausgebildet wie ein überwiegender Teil der Stege 148 in den anpassbaren Bereichen 142.

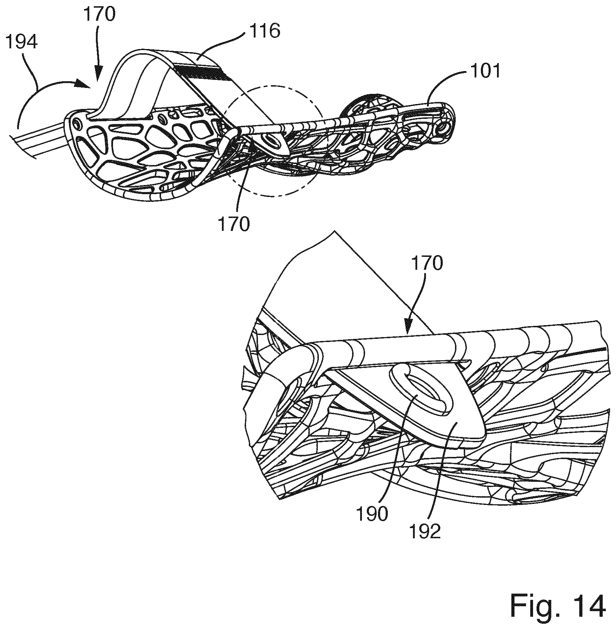

In Figur 14 ist die Durchführung eines Befestigungsgurts 116 durch die Schlitze 170 gezeigt. Der Befestigungsgurt weist an seinem freien Ende 192 einen Ausfädelstop 190 auf. Der Ausfädelstop 190 ist ein mittels Ultraschallschweißens auf dem biegeschlaffen Material des Befestigungsgurts 116 befestigter Ring aus einem Kunststoff. Andere Formen sind ebenso im Sinne der Erfindung. Zur Fixierung des Befestigungsgurts 116 auf der dem Ausfädelstop 190 gegenüberliegenden Seite wird der Befestigungsgurts 116 auf sich selbst zurückgeführt, um die Umrandung des Schlitzes 170 zu erfassen, was durch den Pfeil 194 illustriert ist. Hierzu weist der Befestigungsgurt 116 ein Haken-Ösen-Material (Klettverschluss) auf seiner Oberfläche auf.

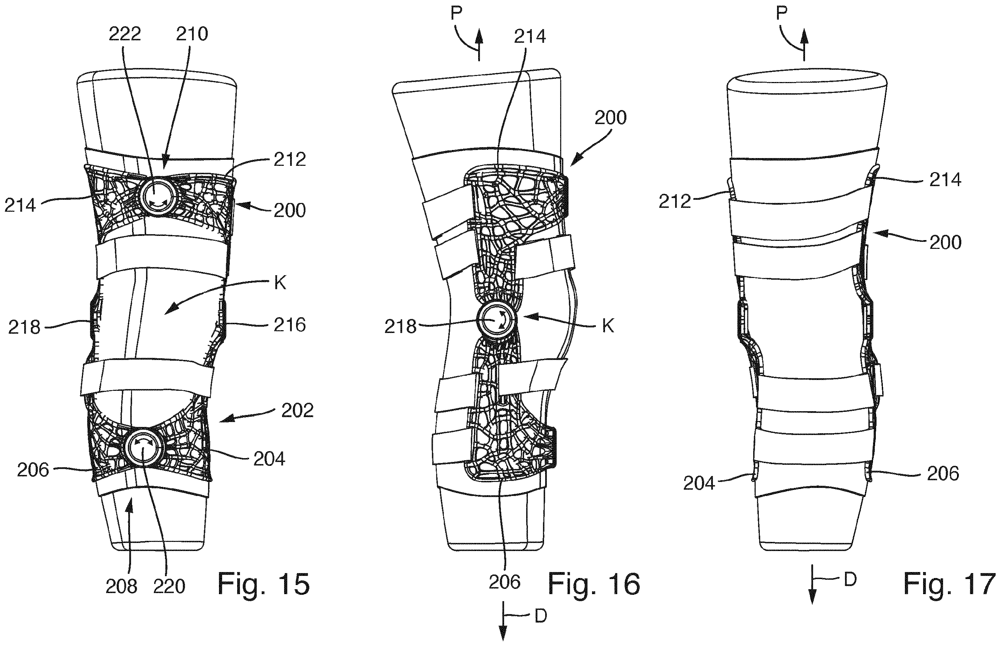

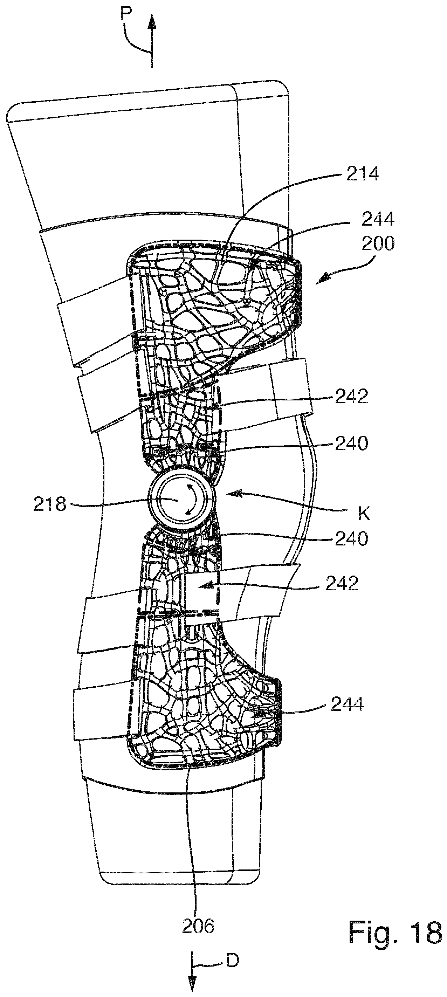

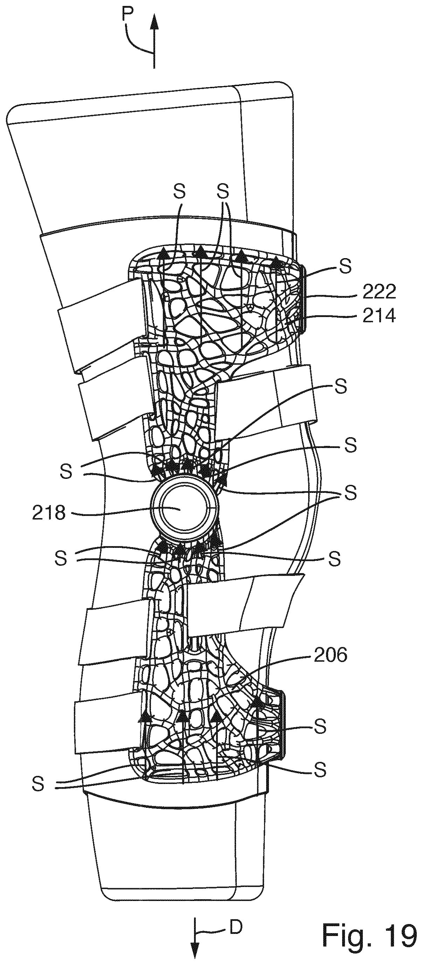

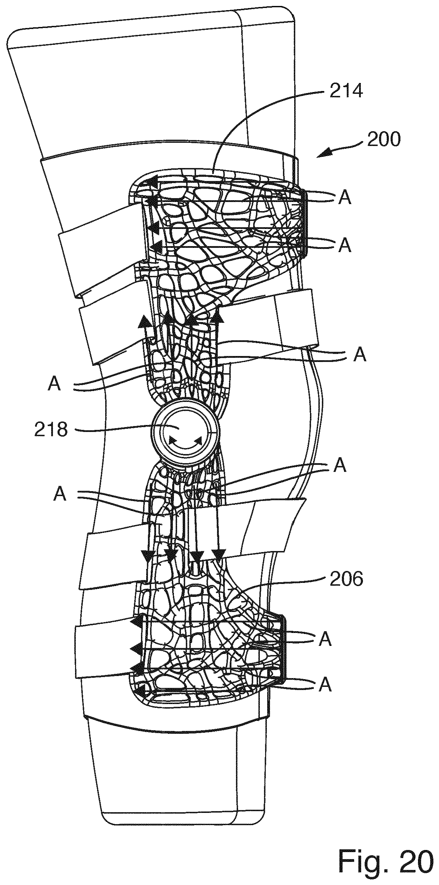

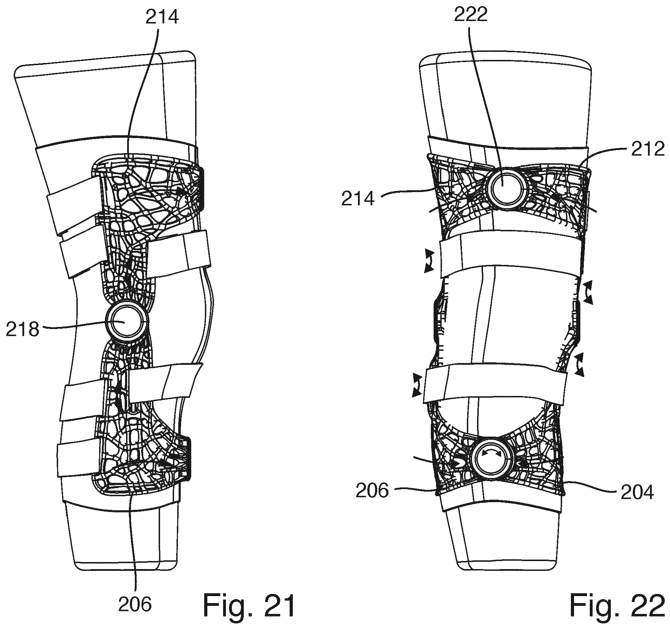

Die Figuren 15-17 zeigen eine Knieorthese 200 in einem angelegten Zustand. In Figur 15 ist die Knieorthese 200 beim Blick auf die Vorderseite des Knies gezeigt. In Figur 16 in einer Seitenansicht und in Figur 17 in einer Rückansicht. In Figur 18 ist die Knieorthese 200 in einer Darstellung entsprechend Figur 16 gezeigt, wobei anpassbare und stabilisierende Bereiche markiert sind. In den Figuren 19 und 20 sind entsprechende Anpassungsrichtungen A und Stabilisierungsrichtungen S eingezeichnet. In den Figuren 21 und 22 ist die Biegsamkeit der anpassbaren Bereiche und die stabilisierende Wirkung der stabilisierenden Bereiche gezeigt.

Die Knieorthese 200 umfasst eine stabilitätsgebende Struktur 202. Die stabilitätsgebende Struktur 202 umfasst eine erste Strukturkomponente 204 sowie eine zweite Strukturkomponente 206, die beide auf einer distalen Seite 208 angeordnet sind. Auf der proximalen Seite 210 der Knieorthese 200 ist eine dritte Strukturkomponente 212 und eine vierte Strukturkomponente 214 angeordnet. Die einzelnen Strukturkomponenten sind über mehrere Gelenke miteinander verbunden.

Auf Höhe der Beugeachse des Kniegelenks K des Trägers der Knieorthese 200 (bezogen auf den vorgesehenen angelegten Zustand der Orthese) sind ein erstes (seitlich angeordnetes) Gelenk 216 und ein zweites (seitlich angeordnetes) Gelenk 218 angeordnet. Diese Gelenke verbinden jeweils eine auf distaler Seite 208 angeordnete Strukturkomponente 204, 206 mit einer auf proximaler Seite angeordneten Strukturkomponente 212, 214.

Die erste Strukturkomponente 204 ist über ein drittes Gelenk 220 mit der zweiten Strukturkomponente 206 verbunden. Die beiden proximalseitig angeordneten Strukturkomponenten, also die dritte Strukturkomponente 212 und die vierte Strukturkomponente 214, sind über ein viertes Gelenk 222 miteinander verbunden.

Das erste Gelenk 216 und das zweite Gelenk 218 ermöglichen eine Beugung des Knies bei angelegter Knieorthese 200. Das dritte Gelenk 220 und das vierte Gelenk 222 ermöglichen es im Zusammenspiel mit der lokalen Flexibilität der stabilitätsgebenden Struktur 202 die Orthese eng am Körper anliegend zu tragen. Insbesondere können das erste (seitlich angeordnete) Gelenk 216 und das zweite (seitlich angeordnete) Gelenk 218 direkt in Kontakt mit dem Knie gebracht werden. Hierzu kann über das dritte Gelenk 220 und das vierte Gelenk 222 die Stellung der jeweils durch sie verbundenen Strukturkomponenten angepasst werden. Das vierte Gelenk 222 erlaubt somit eine Anpassung der Winkelstellung zwischen der dritten Strukturkomponente 212 und der vierten Strukturkomponente 214. Entsprechend ermöglicht das dritte Gelenk 220 eine Anpassung der Stellung der ersten Strukturkomponente 204 gegenüber der zweiten Strukturkomponente 206.

In Figur 18 sind stabilisierende Bereiche 240 sowie anpassbare Bereiche 242 an der zweiten Strukturkomponente 206 und der vierten Strukturkomponente 214 illustriert, die beiden anderen Strukturkomponenten weisen eine entsprechende Anordnung der einzelnen anpassbaren und stabilisierenden Bereiche auf. Die beiden Strukturkomponenten weisen auch jeweils Mischbereiche 244 auf, in denen die Strukturkomponenten einerseits in einer Richtung biegsam und an die Körperform flexibel anpassbar gestaltet sind und andererseits in einer anderen Richtung steif und stabilisierend. Die Stabilisierungsrichtungen S sind in Figur 19 illustriert und die Anpassungsrichtungen A in Figur 20.

In den Figuren 21 und 22 sind die möglichen elastischen Verformungsbewegungen an den Strukturkomponenten illustriert. Von dem Gelenk 218 ausgehend schließt sich in proximaler Richtung ein biegesteifer Bereich an, indem die Orthese starr ausgebildet ist. Weiter in proximaler Richtung P folgt ein anpassbarer Bereich, in dem die Strukturkomponente 214 flexibel ausgebildet ist. Die Flexibilität in diesem Bereich dient zur Kompensation einer Bewegung im vierten Gelenk 222. Auf diesen Bereich folgend ist ein teilweise starrer und teilweise flexibler Bereich 244 angeordnet. In diesem Bereich kann die Strukturkomponente 214 bezüglich ihrer Krümmung, mit der sie um das Bein eines Trägers gelegt wird, angepasst werden. Eine Biegsamkeit in andere Richtungen ist jedoch nicht gegeben. Hierdurch, also durch die Anpassbarkeit an den Umfang des Beins eines Trägers sowie die Flexibilität, die durch das vierte Gelenk 222 bereitgestellt wird und den anpassbaren Bereich 242 kann die Knieorthese 200 sehr eng anliegend am Körper angesetzt angelegt werden. Die Strukturkomponente 206 weist eine entsprechende Abfolge eines steifen bzw. stabilisierenden Bereichs 240 und darauf in distaler Richtung folgend eines anpassbaren Bereichs 242 sowie eines teilweise flexiblen Bereichs 244 auf (Figur 18).

## Patentansprüche

1. Orthese (10, 110, 200) mit einer stabilitätsgebenden Strukturkomponente (18, 20, 101, 102, 103, 204, 206, 212, 214), wobei die stabilitätsgebende Strukturkomponente (18, 20, 101, 102, 103, 204, 206, 212, 214) über ihre gesamte Fläche eine unregelmäßige Gitterstruktur (47, 147) aufweist, wobei die unregelmäßige Gitterstruktur (47, 147) Stege (48, 148) aus festigkeitsgebendem Strukturmaterial aufweist und die Stege (48, 148) unregelmäßig geformte strukturmaterialfreie Aussparungen (52, 152) begrenzen,
wobei die Strukturkomponente (18, 20, 101, 102, 103, 204, 206, 212, 214) flächig ausgebildet ist und
wenigstens eine in der flächigen Erstreckung liegende Stabilisierungsrichtung (S) aufweist, quer zu welcher die Strukturkomponente (18, 20, 101, 102, 103, 204, 206, 212, 214) lokal stabilisierend biegestarr ausgebildet ist, um eine Gliedmaße eines Trägers der Orthese (10, 110, 200) zu stabilisieren, wobei
die Strukturkomponente (18, 20, 101, 102, 103, 204, 206, 212, 214) eine in der flächigen Erstreckung liegende Anpassungsrichtung (A) aufweist, quer zu welcher die Strukturkomponente (18, 20, 101, 102, 103, 204, 206, 212, 214) lokal biegsam ausgebildet ist, so dass sie durch Biegung quer zu Anpassungsrichtung (A) an die Kontur einer Gliedmaße eines Trägers der Orthese (10, 110, 200) in flächige Anlage bringbar ist, wobei
die Orthese (110) eine Handgelenksorthese (110) ist und einen Handgelenksstützabschnitt aufweist, der durch die Strukturkomponente (101) gebildet ist oder Teil der Strukturkomponente (101) ist, wobei die Strukturkomponente (101) einen von proximal nach distal erstreckten stabilisierenden Bereich (140) aufweist, der sich im vorgesehenen angelegten Zustand bis über das Handgelenk erstreckt, wobei beim Blick in distaler Richtung (D) links und rechts des stabilisierenden Bereichs (140) in einer Umfangsrichtung gekrümmte anpassbare Bereiche (142) angeordnet sind, die flexibel an den Armdurchmesser des Trägers anpassbar sind, wobei die Stabilisierungsrichtung (S) in dem stabilisierenden Bereich (140) in proximal-distal-verlaufender Richtung erstreckt ist, und wobei die Anpassungsrichtung (A) der vorhandenen anpassbaren Bereiche (142) in Armumfangsrichtung verläuft bezogen auf den vorgesehenen angelegten Zustand der Orthese, wobei die Strukturkomponente als einstückiges Spritzgussteil hergestellt ist, **dadurch gekennzeichnet, dass** der überwiegende Teil der Stege (48, 148) in dem stabilisierenden Bereich (140) mit einer wenigstens doppelt so dicken Materialstärke (180) ausgebildet ist wie ein überwiegender Teil der Stege (48, 148) in den anpassbaren Bereichen (142),
so dass die Strukturkomponente (101) quer zu der Stabilisierungsrichtung (S) lokal ausreichend steif für ein Stabilisieren eines Körperteils des Trägers ausgebildet ist und im Gegensatz hierzu quer zu der Anpassungsrichtung (A) lokal derart flexibel ausgebildet ist, dass sie an verschiedene Handgrößen bzw. Armdurchmesser anpassbar ist, wobei die lokale Einstellung der Steifigkeit der Strukturkomponente (101) über ihre flächige Erstreckung durch eine gezielte Auswahl der Platzierung und Dimensionierung der einzelnen Stege (48, 148) und eine Variation der Anzahl der Stege (48, 148) erreicht ist, so dass einzelne Bereiche der Strukturkomponente biegesteif und andere Bereiche flexibel gestaltet sind.

2. Orthese (10, 110, 200) nach Anspruch 1, **dadurch gekennzeichnet, dass** die Strukturkomponente (18, 20, 101, 102, 103, 204, 206, 212, 214) mit wenigstens einem Befestigungsgurt (16, 116) verbunden ist, insbesondere der in einer festgelegten Erstreckungsrichtung (174) an der Strukturkomponente (18, 20, 101, 102, 103, 204, 206, 212, 214), insbesondere lösbar, befestigt ist und zur zirkulären Umschlingung der durch die Orthese (10, 110, 200) zu stabilisierenden Gliedmaße des Trägers der Orthese (10, 110, 200) ausgebildet ist, wobei die zirkulären Umschlingung durch den Befestigungsgurt (16, 116) alleine oder durch Befestigungsgurt (16, 116) und wenigstens eine Strukturkomponente (18, 20, 101, 102, 103, 204, 206, 212, 214) der Orthese (10, 110, 200) in Verbindung mit dem Befestigungsgurt (16, 116) erfolgt.

3. Orthese (10, 110, 200) nach einem der vorigen Ansprüche, **dadurch gekennzeichnet, dass** sie zwei Strukturkomponenten (18, 20, 101, 102, 103, 204, 206, 212, 214) umfasst, die jeweils in sich einstückig ausgebildet sind.

4. Orthese (10, 110, 200) nach Anspruch 3, **dadurch gekennzeichnet, dass** die Strukturkomponenten (18, 20, 101, 102, 103, 204, 206, 212, 214) über ein Gelenk (25, 216, 218, 220, 222) schwenkbar miteinander verbunden sind.

5. Orthese (10, 110, 200) nach Anspruch 3 oder 4, **dadurch gekennzeichnet, dass** die Strukturkomponenten (18, 20, 101, 102, 103, 204, 206, 212, 214) über eine Verbindungseinrichtung (20, 120) in zueinander festgelegter Position und Ausrichtung miteinander verbunden sind und gegenüber einander fixiert sind.

6. Orthese (10, 110, 200) nach einem der vorigen Ansprüche, **dadurch gekennzeichnet, dass** eine biegeschlaffe Polsterkomponente (14) auf einer zur Kontaktierung der Gliedmaße des Trägers vorgesehenen Seite der Strukturkomponente (18, 20, 101, 102, 103, 204, 206, 212, 214) angeordnet ist und mit der Strukturkomponente (18, 20, 101, 102, 103, 204, 206, 212, 214) lösbar oder unlösbar verbunden ist.

7. Orthese (110) nach einem der vorigen Ansprüche,
**dadurch gekennzeichnet, dass** sie eine Fingerauflage, die als eine weitere Strukturkomponente (102) ausgebildet ist oder Teil der den Handgelenksstützabschnitt bildenden Strukturkomponente (101) ist, umfasst, wobei die Orthese (110) alternativ oder zusätzlich eine Daumenauflage umfasst, die als eine weitere Strukturkomponente (103) ausgebildet ist oder Teil der den Handgelenksstützabschnitt bildenden Strukturkomponente (101) ist.

## Claims

1. Orthosis (10, 110, 200) comprising a stability-providing structural component (18, 20, 101, 102, 103, 204, 206, 212, 214), wherein the stability-providing structural component (18, 20, 101, 102, 103, 204, 206, 212, 214) has an irregular lattice structure (47, 147) over its entire surface, wherein the irregular lattice structure (47, 147) has struts (48, 148) made of strength-providing structural material and the struts (48, 148) delimit irregularly shaped cutouts (52, 152) that are free of structural material,
wherein the structural component (18, 20, 101, 102, 103, 204, 206, 212, 214) is planar and
has at least one stabilization direction (S) lying in the planar extension, transversely to which direction the structural component (18, 20, 101, 102, 103, 204, 206, 212, 214) is rigid against bending in a locally stabilizing manner, in order to stabilize a limb of a wearer of the orthosis (10, 110, 200), wherein
the structural component (18, 20, 101, 102, 103, 204, 206, 212, 214) has an adaptation direction (A) lying in the planar extension, transversely to which direction the structural component (18, 20, 101, 102, 103, 204, 206, 212, 214) is locally bendable, with the result that it can be brought into surface contact with the contour of a limb of a wearer of the orthosis (10, 110, 200) by bending transversely to the adaptation direction (A), wherein
the orthosis (110) is a wrist orthosis (110) and has a wrist support portion which is formed by the structural component (101) or is part of the structural component (101), wherein the structural component (101) has a stabilizing region (140) extending from proximal to distal, which in the intended applied state extends over the wrist, wherein, when viewed in the distal direction (D), adaptable regions (142) which are curved in a peripheral direction are arranged to the left and right of the stabilizing region (140), which adaptable regions are flexibly adaptable to the diameter of the arm of the wearer, wherein the stabilization direction (S) extends in the proximal-distal direction in the stabilizing region (140), and wherein the adaptation direction (A) of the adaptable regions (142) present runs in the peripheral direction of the arm with respect to the intended applied state of the orthosis, wherein the structural component is manufactured as a one-piece injection-molded part, **characterized in that** the majority of the struts (48, 148) in the stabilizing region (140) are formed with a material thickness (180) at least twice as thick as the majority of the struts (48, 148) in the adaptable regions (142),
with the result that the structural component (101) is locally sufficiently stiff transversely to the stabilization direction (S) to stabilize a body part of the wearer and, in contrast, is locally flexible transversely to the adaptation direction (A) in such a way that it can be adapted to different hand sizes and arm diameters, wherein the local adjustment of the stiffness of the structural component (101) over its planar extension is achieved by a targeted selection of the placement and dimensioning of the individual struts (48, 148) and a variation in the number of struts (48, 148), with the result that individual regions of the structural component are stiff against bending and other regions are flexible.

2. Orthosis (10, 110, 200) according to claim 1, **characterized in that** the structural component (18, 20, 101, 102, 103, 204, 206, 212, 214) is connected to at least one fastening strap (16, 116), in particular which is fastened, in particular detachably, to the structural component (18, 20, 101, 102, 103, 204, 206, 212, 214) in a fixed extension direction (174) and is designed to be circularly wrapped around the limb of the wearer of the orthosis (10, 110, 200) which is to be stabilized by the orthosis (10, 110, 200), wherein the circular wrapping is carried out by the fastening strap (16, 116) by itself or by the fastening strap (16, 116) and at least one structural component (18, 20, 101, 102, 103, 204, 206, 212, 214) of the orthosis (10, 110, 200) in conjunction with the fastening strap (16, 116).

3. Orthosis (10, 110, 200) according to either of the preceding claims,
**characterized in that** it comprises two structural components (18, 20, 101, 102, 103, 204, 206, 212, 214) which are each integrally formed in one piece.

4. Orthosis (10, 110, 200) according to claim 3, **characterized in that** the structural components (18, 20, 101, 102, 103, 204, 206, 212, 214) are pivotably connected to one another via an articulation (25, 216, 218, 220, 222).

5. Orthosis (10, 110, 200) according to claim 3 or 4, **characterized in that** the structural components (18, 20, 101, 102, 103, 204, 206, 212, 214) are connected to one another in a fixed relative position and orientation via a connecting device (20, 120) and are secured in relation to one another.

6. Orthosis (10, 110, 200) according to any of the preceding claims,
**characterized in that** a pliable padding component (14) is arranged on a side of the structural component (18, 20, 101, 102, 103, 204, 206, 212, 214) which is provided for contacting the limb of the wearer, and is detachably or non-detachably connected to the structural component (18, 20, 101, 102, 103, 204, 206, 212, 214).

7. Orthosis (110) according to any of the preceding claims, **characterized in that** it comprises a finger rest which is formed as a further structural component (102) or is part of the structural component (101) forming the wrist support portion, wherein the orthosis (110) alternatively or additionally comprises a thumb rest which is formed as a further structural component (103) or is part of the structural component (101) forming the wrist support portion.

## Revendications

1. Orthèse (10, 110, 200) avec un composant structurel stabilisant (18, 20, 101, 102, 103, 204, 206, 212, 214), lequel présente sur toute sa surface une structure de treillis irrégulière (47, 147), ladite structure de treillis irrégulière (47, 147) comportant des nervures (48, 148) en matériau structurel conférant la résistance et les nervures (48, 148) délimitant des évidements dépourvus de matériau structurel de forme irrégulière (52, 152),
où le composant structurel (18, 20, 101, 102, 103, 204, 206, 212, 214) est réalisé en surface et
présente au moins une direction de stabilisation (S) s'étendant dans la surface, par rapport à laquelle le composant structurel (18, 20, 101, 102, 103, 204, 206, 212, 214) est localement rigide à la flexion afin de stabiliser un membre d'un porteur de l'orthèse (10, 110, 200), où
le composant structurel (18, 20, 101, 102, 103, 204, 206, 212, 214) présente une direction d'adaptation (A) s'étendant dans la surface, perpendiculairement à laquelle le composant structurel (18, 20, 101, 102, 103, 204, 206, 212, 214) est localement flexible à la flexion, de sorte que, par flexion perpendiculairement à la direction d'adaptation (A), il puisse être mis en contact en surface avec le profil d'un membre d'un porteur de l'orthèse (10, 110, 200), l'orthèse (110) étant une orthèse de poignet (110) et comprenant une section de maintien du poignet formée par le composant structurel (101) ou faisant partie du composant structurel (101), le composant structurel (101) présentant une zone stabilisante (140) s'étendant de proximal à distal et qui, dans l'état prévu en position appliquée, s'étend au-delà du poignet, où, regardant en direction distale (D), des zones adaptables (142) incurvées en direction circonférentielle sont disposées à gauche et à droite de la zone stabilisante (140) et sont adaptables de manière flexible au diamètre du bras du porteur, la direction de stabilisation (S) s'étendant dans la zone stabilisante (140) en direction proximodistale, et la direction d'adaptation (A) des zones adaptables (142) existantes s'étendant en direction du tour de bras par rapport à l'état prévu d'application de l'orthèse, le composant structurel étant fabriqué comme une pièce moulée par injection monobloc, **caractérisé en ce que** la majeure partie des nervures (48, 148) dans la zone stabilisante (140) est réalisée avec une épaisseur de matériau (180) au moins deux fois supérieure à celle de la majeure partie des nervures (48, 148) dans les zones adaptables (142), de sorte que le composant structurel (101) soit localement suffisamment rigide, perpendiculairement à la direction de stabilisation (S), pour stabiliser un segment corporel du porteur et, en revanche, localement si flexible perpendiculairement à la direction d'adaptation (A) qu'il puisse s'adapter à différentes tailles de main ou différents diamètres de bras, l'ajustement local de la rigidité du composant structurel (101) sur son étendue de surface étant obtenu par une sélection ciblée du placement et des dimensions des nervures individuelles (48, 148) et par une variation du nombre de nervures (48, 148), de sorte que certaines zones du composant structurel sont rigides à la flexion et d'autres zones sont conçues flexibles.

2. Orthèse (10, 110, 200) selon la revendication 1, **caractérisée en ce que** le composant structurel (18, 20, 101, 102, 103, 204, 206, 212, 214) est relié à au moins une sangle de fixation (16, 116), en particulier fixée au composant structurel dans une direction d'étendue déterminée (174), notamment de façon amovible, et conçue pour l'enveloppement circulaire du membre du porteur à stabiliser par l'orthèse (10, 110, 200), la dite enveloppe circulaire étant réalisée par la sangle de fixation (16, 116) seule ou par la sangle de fixation (16, 116) et au moins un composant structurel (18, 20, 101, 102, 103, 204, 206, 212, 214) de l'orthèse (10, 110, 200) en combinaison avec la sangle de fixation (16, 116).

3. Orthèse (10, 110, 200) selon l'une quelconque des revendications précédentes, **caractérisée en ce qu'**elle comprend deux composants structurels (18, 20, 101, 102, 103, 204, 206, 212, 214) chacun formé en une seule pièce.

4. Orthèse (10, 110, 200) selon la revendication 3, **caractérisée en ce que** les composants structurels (18, 20, 101, 102, 103, 204, 206, 212, 214) sont reliés entre eux de façon pivotante au moyen d'une articulation (25, 216, 218, 220, 222).

5. Orthèse (10, 110, 200) selon la revendication 3 ou 4, **caractérisée en ce que** les composants structurels (18, 20, 101, 102, 103, 204, 206, 212, 214) sont reliés l'un à l'autre au moyen d'un dispositif de connexion (20, 120) dans une position et une orientation déterminées l'un par rapport à l'autre et sont fixés l'un par rapport à l'autre.

6. Orthèse (10, 110, 200) selon l'une quelconque des revendications précédentes, **caractérisée en ce qu'**un composant de rembourrage souple (14) est disposé sur une face du composant structurel (18, 20, 101, 102, 103, 204, 206, 212, 214) destinée à entrer en contact avec le membre du porteur et est relié au composant structurel (18, 20, 101, 102, 103, 204, 206, 212, 214) de manière amovible ou non amovible.

7. Orthèse (110) selon l'une des revendications précédentes, **caractérisée en ce qu'**elle comprend un appui pour les doigts, formé comme un autre composant structurel (102) ou faisant partie du composant structurel (101) formant la section de maintien du poignet, l'orthèse (110) comprenant, alternativement ou en supplément, un appui pour le pouce, formé comme un autre composant structurel (103) ou faisant partie du composant structurel (101) formant la section de maintien du poignet.
